# EUROPEAN PATENT APPLICATION

(11) **EP 3 098 304 A1**
(43) Date of publication of application: **30.11.2016**
(21) Application number: 15169593.9
(22) Date of filing: 28.05.2015
(51) Int. Cl.: C12N 5/00, C12M 1/22, C12M 1/32, C12M 1/12, C12N 5/0775

(54) **HYBRID CELL DETACHMENT-FREE CELL EXPANSION SYSTEM FOR ADHERENT CELL CULTURING AND RELATED PROCEEDING PROTOCOL**

(71) Applicant: Fraunhofer-Gesellschaft zur Förderung der angewandten Forschung e.V., 80686 München (DE)
(72) Inventor: Confalonrieri, Davide, 97070 Würzburg (DE); Hansmann, Jan, 97246 Eibelstadt (DE); Walles, Heike, 97082 Würzburg (DE); Krziminski, Steffan, 97080 Würzburg (DE)
(74) Representative: Schrell, Andreas

(57) **Abstract**

The present invention relates to a hybrid cell culturing system, a process for culturing cells using said hybrid cell culturing system, a cell culture device comprising said hybrid cell culturing system and use of at least one second cell culturing support designed to be arranged to at least one arrangement position of a first cell culturing support for culturing cells.

## Description

The present invention relates to a hybrid cell culturing system, a process for culturing cells using said hybrid cell culturing system, a cell culture device comprising said hybrid cell culturing system and use of at least one second cell culturing support designed to be arranged to at least one arrangement position of a first cell culturing support for culturing cells.

In the last thirty years the medical scenery acknowledged and encouraged the growth and development of a multitude of new branches of the basic and applied sciences intended to change the paradigms of classical medicine and shift the attention of the clinical efforts on cellular therapies rather than on pharmacology. New approaches on cellular therapies opened the road to Tissue Engineering and Regenerative Medicine, a new branch of applied sciences which is gaining more and more importance in clinical applications. Though a critical amount of cells is required for therapeutic applications, currently available cell expansion systems (CESs), used to expand and differentiate cells (US 5,162,225 and US 6,001,585), face important limitations. In fact, while several parameters have to be kept under tight control during cell expansion, it appears that only surface availability and cell doubling time are able to define the final cell yield of an expansion system. Most of the commercially available systems try to avoid cell-cell contact growth inhibition, which can ultimately lead to cell senescence - growth arrest and cell death, by increasing the culturing surface. Expansion of the available culturing surface is generally achieved by enzymatic, chemical or physical detachment of all the cultured cells from the corresponding original culturing surface and reseeding upon dilution on a larger surface, meaning either a single broader surface or multiple surfaces of which the sum of final dimensions is bigger than the original culturing surface. Such treatments ultimately lead to important re-modeling of the cell cytoskeleton, that transiently blocks or delays cell proliferation and alters protein expression, with relevant consequences on the long term on proliferation and differentiation potential, cell morphology and immune phenotype, genomic stability and ageing. One of the most common methods involves extensive cell passaging after enzymatic cell detachment. However, enzymatic cell passaging can lead to genotoxic mutations and ultimately decrease cell self-renewal and differentiation potentials, especially in adult primary cultures (Vacanti et al., Phenotypic changes of adult porcine mesenchymal stem cells induced by prolonged passaging in culture, Journal of Cell Physiol., 2005, 205(2), pages 194 to 201). Other common systems are based on the employment of different types of micro carriers (Feng et al., A scalable approach to obtain mesenchymal stem cells with osteogenic potency on apatite microcarriers, Journal of Biomater Appl., 2013, 29(1), pages 93 to 103). Moreover, exposure to shear stress due to the micro carrier dynamic culturing conditions could lead to early cell differentiation and impairment of the overall proliferation potential (Tseng et al., Spontaneous osteogenesis of MSCs cultured on 3D microcarriers through alteration of cytoskeletal tension, Biomaterials, 2012, 33(2), pages 556 to 564).

Many cell expansion systems (CESs) are currently used for adherent cell culturing, comprising hollow fibers bioreactors, cultures on micro carriers in spinner flasks and various 2D expansion systems among whom T-Flasks represent the most widely used option in laboratory practice. All the existing CESs try to solve the problem of expanding the available cell culturing surface, trying to avoid the limitations of 2D T-Flask cultures related to limited culturing surface, lack of perfusion or dynamic culturing conditions and need of cell detachment for passaging. The only exception encountered so far is a patent application (CA2843001 A1) disclosing a mesenchymal progenitor cells (MPCs) expansion without the standard plastic-adherence isolation step and providing an expanded population of human mesenchymal progenitor cells which result from the non-static suspension culturing of an input population enriched for non-hematopoietic progenitor cells. However, though the results of this method may result questionable, it cannot be applied so far to other adherent cell types. One of the more widely used culturing methods comprises the use of floating micro carriers to be cultured in spinner flasks (Frauenschuh et al., A microcarrier-based cultivation system for expansion of primary mesenchymal stem cells, Biotechnology Prog., 2007, 23(1), pages 187 to 193) or in plastic bag bioreactors (US 2013/236970 A1 and US 2007/0264713 A1). Variants of this method can be adapted under specific needs, as for the Wave Bioreactor for influenza virus production (Genzel et al., Wave microcarrier cultivation of MDCK cells for influenza virus production in serum containing and serum-free media, Vaccine, 2006, 24(35-36), pages 6074 to 6087).

Although the recent development of non-fixed floating spherical micro carriers is acquiring relevance for adherent cell culturing, the use of such micro carriers alone meets several limitations among whom a significant cell loss during seeding, thus making this systems prohibitive for primary cells expansions or cell expansion starting from a limited cell number, and poor control of the shear stress on the cells, thus affecting the cell proliferation-differentiation balance. Furthermore, spinner flasks are not disposable systems, bringing in practical problems related to cleaning, sterilization and maintenance. Finally, while cell migration from colonized floating micro carriers to empty floating micro carriers under static or dynamic conditions could represent a good method for scalable cell expansion, this system suffers the same limitations previously reported, and ultimately leads to the formation of micro carrier clumps (Feng et al., A scalable approach to obtain mesenchymal stem cells with osteogenic potency on apatite microcarriers, Journal of Biomater Appl., 2013, 29(1), pages 93 to 103) generally not suitable for injection or other clinical applications.

Other CESs for adherent cells exploit 3D conditions, such as encapsulating cells within a semipermeable membrane and then suspending the capsules in growth medium (US 4,495,288), and culturing cells on a three dimensional matrix like collagen (US 4,963,489). However, all these methods depend on surface availability or rely on cell detachment for expansion.

Another important CES relies on cell culturing on hollow fiber cartridges, allowing medium exchange, perfusion and collection of secreted molecules (US 3,821,087; US 3,883,393; US 4,220,725; US 4,391,912; US 5,763,261). Also this system depends on surface availability, and cells may undergo significant changes after confluence is reached.

Finally, different CESs rely on 2D culturing surfaces only, by expanding the available culturing surface on different compartments. Cell culture plates have been developed to have two or more open-topped wells of increasing area and volume (US 6,410,310), allowing lateral diffusion of molecules across a barrier membrane separating communicating diffusion wells (US 5,578,490; US 5,972,694), or hosting several stacked culturing planes (CA 2679160 A1). These cell culture plates allow cell expansion without significant cell manipulation, but are still limited in cell yield by the available surface of the plate and depend on cell detachment for passaging. Another 2D CES rely on the use of a highly elastic culture surface that can be enlarged up to 800% of the initial surface area over the course of a 20-day culture. Also in this case the final cell yield depends on the elasticity of the material and the final area covered by the culturing surface, and cell detachment is ultimately required to further passage the cells. Furthermore, US 5,139,946 discloses a device comprising an upper and a lower polymeric film layer and a gas and liquid permeable flow divider membrane.

Taken together, there is a strong need for a new detachment-free and shear stress-free cell expansion system able to allow rapid production of Tissue Engineering products starting from minimal, preferably explanted, samples.

Thus, the problem underlying the present invention is the provision of a culturing method or a culturing device wherein the culturing of cells, preferably adherent cells, is improved. Especially, a cultivation process and a device with which said process can be performed should be provided, wherein cells, preferably adherent cells, can be transferred from a first culturing device to a second culturing device without being detached from the surface of the first culturing device.

Said technical problem is in particular solved by the independent claims of the present invention.

The present invention relates to a hybrid cell culturing system, preferably for cultivation of cells, preferably of adherent cells, comprising:
i) a first cell culturing support fixable in a cell culture device, which first cell culturing support comprises a plurality of arrangement positions, and
ii) at least one second cell culturing support designated to be arranged to at least one arrangement position of the first cell culturing support, which at least one second cell culturing support is removable from the at least one arrangement position of the first cell culturing support, preferably during or after the cultivation of the cells, preferably of the adherent cells.

Under the term "planar support" a support is understood which has spatial expansions x', y' and z' along spatial axes x, y and z in a three dimensional space, wherein the spatial expansion x' and y' are distinctly higher than the spatial expansion z', preferably at least by the factor 5, preferably at least by the factor 10, preferably at least by the factor 50, preferably at least by the factor 100, preferably at least by the factor 1000. Accordingly, the planar support has a spatial expansion in all of the three spatial axes.

In context with the present invention, the term "adherent cells" refers to cells of the type which can be cultured in a cell culture medium on the first and second cell culturing support, which are preferably inert, as a monolayer or as a multilayer, but in particular as a monolayer. The adherent cells contact the surface of the first and second cell culturing support and adhere thereto. At their surface, the adherent cells therefore have preferably points of contact with the, preferably inert, surface of the first or second cell culturing support. In this case, the adherent cells do not grow with their entire area on the first and second cell culturing support, but form a bond to the surface of the first and second cell culturing support via what are known in the art as cell branches which form this contact to the first and second cell culturing support. Adherent cells usually form a continuous cell layer. Adherent cells often display a density-dependent proliferation inhibition, also referred to as contact inhibition, which can occur when the confluence is exceeded. Adherent cells often derive from tissues, for example skin, muscles, nerves, the liver and kidneys. Examples of adherent cells are fibroblasts, HeLa cells and many tumour cells. The culture media which are known to the person skilled in the art and are selected depending on the type of cell to be grown are suitable as the cell culture medium for cultivation of the adherent cells in accordance with the present invention. According to the invention, the adherent cells are preferably eukaryotic cells, in particular mammalian cells. According to the invention, the cells are preferably animal cells. According to the invention, the cells are preferably mammalian cells, in particular human, bovine or murine cells. Alternatively, it is also possible to cultivate prokaryotic cells, plant cells or fungal cells, preferably provided that they are adherent cells.

In context with the present invention, the term "support fixable in a cell culture device" refers to a support which can be fixed in a cell culture device. By said fixation the support is connected to the cell culture device in a manner that it is locked in the cell culture device and cannot be removed by a method used for the removal of the at least one second cell culturing support.

In context with the present invention, the term "a removable support" refers to a support which can be separated from another support, preferably by a mechanical and/or a physical method. Accordingly, the second cell culturing support can be removed from the first cell culturing support by different removal methods which are able to reverse the arrangement procedure. The at least one second cell culturing support can be arranged to, preferably positioned at, the at least one arrangement position by gravity force, magnetic forces, preferably electromagnetic induction or superparamagnetism, friction force and/or intermolecular forces. Accordingly, the removal procedure is preferably selected from at least one or exactly one of removing a magnet, demagnetizing the at least one second cell culturing support, supplying fluid, applying ultrasound and/or vibrations, knocking, shaking and agitating.

In context with the present invention, the term "arrangement position" refers to an opening or recess which partially or completely, preferably completely, extends from one surface of the first cell culturing support to another, preferably opposite, surface of the first cell culturing support. The arrangement position is preferably a concavity, a nice or a pore. The arrangement position can be provided by etching, freeze drying, mechanical removal methods, preferably drilling, moulding, preferably injection moulding, stereolithography, 3D printing and electrospinning. The arrangement position has an aperture in at least one, preferably at least two, preferably exactly one or two, different, preferably opposite, surface areas of the first cell culturing support, being a planar or three dimensional support.

The hybrid cell culturing system according to the present invention comprises a first cell culturing support which can be fixed in a cell culture device. Thus, in a preferred embodiment of the present invention, the first cell culturing support can be fixed in the cell culture device so that it cannot be removed, preferably without destructing the cell culture device. Furthermore, the hybrid cell culturing system comprises at least one second cell culturing support which is designed to be arranged, preferably designed to be positioned or immobilized, to at least one arrangement position of the first cell culturing support. The at least one second cell culturing support is preferably removable and replaceable from the first cell culturing support.

By the subject-matter of the independent claims, preferably by the hybrid cell culturing system and the process for culturing cells, means are preferably provided for a cell detachment-free culturing and passaging of adherent cell cultures. Preferably, it is possible to modify the hybrid cell culturing system and the method for culturing cells using said hybrid cell culturing system in several different ways by adapting it to the intended use. By the hybrid cell culturing system according to the present invention, preferably adherent, cells can be expanded without cell detachment from the culturing surface, that is the cells can be transferred from a first culturing device to a second culturing device without being detached from the surface of the at least one second cell culturing support which can easily be removed from the first cell culturing support and used as carrier of the cells, preferably adherent cells, which can be used as a starting point of further cultivation of said cells in another cell culture device. The hybrid cell culturing system is advantageous over a method, wherein the available culturing surface for adhering cells is increased in order to avoid a cell contact growth inhibition and therefore minimizing cell loss. Cell expansion on two-dimensional surfaces according to the state of the art requires cell detachment through enzymatic, chemical or physical treatments on their original surface, thereby reassembling and remodeling of the cytoskeleton is enforced which ultimately leads to loss of differentiation proliferation potential, genomic mutations, cell loss during passaging and premature ageing in the methods according to the prior art. The hybrid cell culturing system according to the present invention, in contrast thereto, allows passaging of a part of the cultured cells, leaving space for the remaining cells for further proliferation. Especially, by said hybrid cell culturing system the cell detachment from their original culturing surface can be avoided, thereby avoiding massive cytoskeleton remodeling and consequent problems. Furthermore, the at least one second cell culturing support provides the cells with a larger culturing surface compared to two-dimensional culture systems. In sum, a detachment-free and shear stress-free cell expansion system is preferably provided allowing rapid production of Tissue Engineering products starting from minimal explanted samples. By the hybrid cell culturing system preferably a "partial passaging" concept without detachment of adherent cells can be realized.

The first cell culturing support is advantageously intended to host the cells during the initial phase(s) of the expansion, thus minimizing cell loss during seeding and allowing dramatically lower starting cell densities. Thus, the hybrid cell culturing system according to the present invention provides a significant advantage over cell expansion known from the prior art. It can also be tuned to better fit physiological culturing conditions, namely by the variation of the structure of the first cell culturing support and the at least one second cell culturing support, which allows the cell culturing in 3D conditions. Thus, also 3D scaffolds can be used for cell expansion purposes and eventually co-culturing of two different cell types in different compartments can be performed in order to boost cell cell-signaling driven cell proliferation. Especially, by the hybrid cell culturing system the shear stress imposed on the cell types cultured therein can be reduced, by either culturing them on static conditions or allowing automatic medium exchange.

The present invention preferably relates to a hybrid cell culture system, wherein the shape and dimension of the at least one second cell culturing support allows the at least one second cell culturing support to be arranged at least partially in or on the at least one arrangement position of the first cell culturing support.

The shape and dimension of the at least one second cell culturing support allows the at least one second cell culturing support to be arranged properly, preferably at least partially, preferably completely within the at least one arrangement position of the first cell culturing support, that is preferably being embedded or hosted therein. The at least one second cell culturing support can have different geometries. The at least one second cell culturing support is preferably degradable in order to isolate the cells after expansion. In an alternatively preferred embodiment of the present invention, the at least one second cell culturing support is non-degradable, preferably when used as scaffold, preferably for tissue engineering applications.

Preferably, the at least one second cell culturing support is designed to be arranged to exactly one arrangement position of the first cell culturing support. Thus, in this preferred embodiment, exactly one second cell culturing support has preferably the shape and dimension to allow its arrangement to exactly one arrangement position of the first cell culturing support.

The present invention preferably relates to a hybrid cell culturing system, wherein the arrangement positions of the first cell culturing support have an average diameter from 1 to 550 µm, preferably 90 to 550 µm, preferably 100 to 500 µm, preferably 150 to 400 µm, preferably 200 to 300 µm.

The present invention preferably relates to a hybrid cell culturing system, wherein the at least one second cell culturing support is a microparticle. Preferably, the at least one second cell culturing support is a collagen microbead(s) with an, preferably average, diameter of 1 to 550 µm, preferably 100 to 500 µm, preferably 150 to 200 µm.

The present invention preferably relates to a hybrid cell culturing system, wherein the at least one cell culturing support, preferably the at least one microparticle, has a diameter from 1 to 600 µm, preferably from 90 to 600 µm, preferably 100 to 550 µm, preferably 150 to 450 µm, preferably 150 to 200 µm, preferably 200 to 350 µm.

Preferably, the minimal distance between two adjacent arrangement positions of the first cell culturing support is at least 20 µm, preferably at least 30 µm, preferably at least 40 µm, preferably at least 50 µm, preferably at least 100 µm, preferably at least 150 µm, preferably at most 400 µm, preferably at most 300 µm, preferably at most 200 µm, preferably from 40 to 400 µm, preferably from 50 to 300 µm, preferably from 100 to 200 µm.

The present invention preferably relates to a hybrid cell culturing system, wherein the first cell culturing support is characterized by a spacing between the arrangement positions' central axis, which is preferably at least 10%, preferably at least 50%, preferably at least 100%, preferably at least 150%, preferably at least 200%, preferably at least 250%, preferably at most 400%, preferably at most 300% of the average arrangement position diameter. Said spacing should avoid contact and clumping of the at least one second, preferably spherical, cell culturing support.

Preferably, the spacing or distance between the two geometrical centers of the surface area of two adjacent arrangement positions is at least 100 µm, preferably at least 150 µm, preferably at least 200 µm, preferably at least 300 µm, preferably at most 500 µm, preferably at most 400 µm. Preferably, the surface area of the arrangement positions is used for determining the geometrical center which is faced towards the at least one second cell culturing support, preferably which is faced towards a cell culture medium used during the cultivation of cells. The distance or spacing between the longitudinal axes of two adjacent arrangement positions is preferably at least 110%, preferably at least 120%, preferably at least 130%, preferably at least 150%, preferably at least 200%, preferably at least 250%, preferably at most 400%, preferably at most 300% of the average arrangement position diameter thereof.

Preferably, the arrangement position spacing is adjusted to fit any desired specification according to the need.

In context with the present invention the "longitudinal axis of an arrangement position" is preferably perpendicular to the tangent of the geometrical center of the surface area of the arrangement position and goes through said geometrical center of surface area wherein preferably the surface area of the arrangement position is used for determining the geometrical center which is faced towards the at least one second cell culturing support, preferably which is faced towards a cell culture medium used during the cultivation of cells.

Preferably, the average arrangement position size diameter of the planar first cell culturing support is at most 5 to 10% smaller than the average diameter of the at least one second cell culturing support, in order to block the at least one second cell culturing support on the first cell culturing support as "particles on a sieve". Preferably, the average arrangement position size diameter of the three-dimensional first cell culturing support is at most 5 to 10% greater than the average diameter of the at least one second cell culturing support, in order to facilitate migration of the cells from the hosting surface to the at least one second cell culturing support.

Preferably, the cross section, preferably of the surface area, of the arrangement positions, is circular, oval, polygonal with, preferably three to 12 edges, preferably 3 to 6 edges, trigonal, rectangular, pentagonal, hexagonal, tapered, elliptical, star shaped, square shaped or shapeless. Preferably, the cross section, preferably of the surface area, of the arrangement positions are different in shape, preferably their shape is selected from circular, oval, polygonal with, preferably three to 12 edges, preferably 3 to 6 edges, trigonal, rectangular, pentagonal, hexagonal, tapered, elliptical, star shaped, square shaped and shapeless shape. Preferably, the cross section of the at least one second cell culturing support is identical to the cross section of the arrangement positions of the first cell culturing support. The cross section of the at least one second cell culturing support is circular, oval, polygonal with, preferably three to 12 edges, preferably three to 6 edges, rectangular, tapered, elliptical, star shaped, square shaped, triangular, pentagonal, hexagonal or shapeless.

Preferably, the hybrid cell culturing system comprises as many second cell culturing supports as 50%, preferably at least 50%, preferably 60%, preferably at least 60%, preferably at least 70%, preferably at least 80%, preferably at least 90%, preferably at least 95%, preferably at least 99%, preferably 100% of the arrangement positions comprised in the first cell culturing support.

Preferably, the average diameter of the at least one first cell culturing support is preferably at least 90%, preferably at least 95%, preferably at least 100%, preferably at least 110%, preferably at most 95%, preferably at most 100%, preferably at most 105%, preferably at most 110%, preferably at most 120% of the average diameter of the arrangement positions of the first cell culturing support.

Preferably, the first and/or the at least one second cell culturing support is composed of an inert material. The term 'inert material' means that said material allows the attachment of adherent cells but is not toxic or detrimental for the cultivation of cells, preferably of adherent cells. Preferably, the inert material is glass, silica, titanium oxide, aluminum oxide, a metal, a metal alloy, a polymer or a hydrogel. Preferably, the polymer is swellable, preferably water swellable. Preferably, the polymer is a cross linked polymer.

The arrangement positions of the first cell culturing support have preferably at least two, preferably exactly two openings, namely one opening in a first surface area of the first cell culturing support and the second opening in a second surface area of the first cell culturing support, wherein the first surface area and the second surface area are preferably present on opposite sides of the first cell culturing support. By said specific design of the arrangement positions it is possible to arrange to the first surface of the first cell culturing support the at least second cell culturing support which can be, subsequently, removed from the first cell culturing support by a removal method, preferably as mentioned above, preferably by supplying fluid, preferably a liquid or a gas, preferably air, through the second opening of the first cell culturing support. Preferably, by a first cell culturing support comprising arrangement positions with two openings on different surfaces of the first cell culturing support at least two, preferably exactly two, different types of, preferably adherent, cell can be cultured.

The present invention preferably relates to a hybrid cell culturing system, wherein the first cell culturing support is a planar or three-dimensional support. The flexibility of the planar support is to be determined according to the fixing properties of a surrounding holding device of a cell culture device, properly designed to keep the culturing surface of the planar support in place. The thickness of the planar support is preferably only functional to the culturing surface stability upon fixation in this device. Preferably, a three-dimensional support is used whose final dimensions are to be determined according to the arrangement position dimensions, number, spacing between arrangement positions and any additional requirement needed for the fixation of the three-dimensional support to a surrounding device. In this case, the structure of the three-dimensional support should be properly designed so that the whole culturing surface of the three-dimensional support is properly accessible to the culture medium in order to allow nutrient exchange for cell survival and proliferation, and additional arrangement positions may then be required for such purpose. Preferably, the mechanical properties of the first cell culturing support are to be determined to withstand the incident pressure later used to remove the at least one second cell culturing support and to properly fit the requirements for its fixation into the cell culture device. Preferably, the stiffness and functionalization of the first and at least one second cell culturing support are to be matched to the requirements of the desired cell type, thus optimizing cell proliferation and attachment.

The culturing surface of the at least one second cell culturing support is preferably a scaffold capable of supporting secondary cell attachment, growth and proliferation in a 2D or 3D environment after cell migration from the first cell culturing support. The at least one second cell culturing support can be represented by a porous or non-porous scaffold of generally small dimensions when compared to the first cell culturing support, and should be preferably characterized by a shape that fits as best as possible the shape of the corresponding arrangement positions on the first cell culturing support, and by dimensions that allow it to be arranged to, preferably hosted on the arrangement positions of first cell culturing support. Preferably, a large extent of the surfaces of the first cell culturing support and the at least one second cell culturing support are in contact with each other in order to better allow cell migration from the first cell culturing support to the at least one second cell culturing support.

The scaffold constituting the at least one second cell culturing support should preferably be able to support cell proliferation after transferring in a secondary expansion system, allowing proper cell attachment as well as gas and nutrient diffusion into the scaffold.

The present invention preferably relates to a hybrid cell culturing system, wherein the planar support is a petri dish, a plate or membrane. Preferably, the membrane is a biological membrane, preferably an animal-derived membrane produced by removing the serosa membrane and the mucosa layer from a decellularized tract of porcine intestine.

According to the invention, the membrane is preferably part of a membrane unit. According to the invention, the membrane unit preferably consists of a membrane and a frame. Alternatively, the membrane unit can consist only of a membrane. The person skilled in the art is familiar with suitable membranes which are suitable for cultivating cells, in particular adherent cells.

According to the invention, the membrane is preferably selected from the group consisting of polyethylene terephthalate (PET) membranes, polycarbonate (PC) membranes, nylon membranes, amphoteric nylon membranes, positively charged nylon membranes, negatively charged nylon membranes, polytetrafluoroethylene (PTFE) membranes, cellulose ester membranes, cellulose acetate membranes, cellulose nitrate membranes, cellulose mixed ester membranes, regenerated cellulose membranes. The membrane has by definition a porous structure, so that liquids can be exchanged via the membrane.

The present invention preferably relates to a hybrid cell culturing system, wherein the three-dimensional support is a polymeric scaffold, an organ or a tissue. The three-dimensional support is preferably of cylindrical shape.

The present invention preferably relates to a hybrid cell culturing system, wherein the at least one second cell culturing support is porous or non-porous.

The present invention also relates to a process for culturing cells, preferably adherent cells, using the hybrid cell culturing system in accordance with the present invention.

The present invention also relates to a process for culturing cells, preferably adherent cells, comprising the following process steps:
a) arranging at least one second cell culturing support to at least one arrangement position of a plurality of arrangement positions of a first cell culturing support, as to obtain a hybrid cell culturing system, preferably in accordance with one or more embodiments of the present invention, and
b) culturing, preferably expanding or colonizing, cells on the hybrid cell culturing system.

Preferably, cells are comprised on, preferably attached to, the at least one second cell culturing support provided in step a). Preferably, cells are comprised on, preferably attached to, a first surface of the first cell culturing support provided in step a). Said first surface of the first cell culturing support is faced, preferably upwardly, towards the cell culture medium used for culturing the cells on the hybrid cell culturing system. Preferably, in step a) the at least one second cell culturing support and the first cell culturing support is cell free, preferably adherent cell free, and before the process step b) is performed, cells to be cultured are seeded on the hybrid cell culturing system prepared in step a).

The first cell culturing support preferably has two surfaces, in particular if it is sheet-shaped or disc-shaped, for example if it is a membrane, namely an upper side and a lower side. The hybrid cell culturing system can however also have more than two surface areas, for example if it is thicker than a membrane, for example if it is sponge-shaped. According to the invention, preferably only one surface of the hybrid cell culturing system serves as the growth area of the cells. If the first cell culturing support is used, in accordance with the invention, preferably horizontally, then according to the invention preferably the upper surface, i.e. the upper side, of the first cell culturing support serves as the growth area of the cells.

Preferably, in the method according to the invention the, preferably adherent, cells to be cultured are solely applied to one of the at least two surface areas of the first cell culturing support. On an opposite surface area a different cell type is preferably cultured, which preferably serve as feeder cells for the cells to be cultured.

The cells on the hybrid cell culturing system, preferably first and/or second cell culturing support, can be cultivated in the conventional manner. The person skilled in the art is familiar with a broad range of cultivation methods for, preferably adherent, cells, from which he can select suitable methods.

The present invention preferably relates to a process, wherein after step b) the following process steps are performed:
c) removing the at least one second cell culturing support from the first cell culturing support,
d) arranging at least one further, preferably cell free, preferably adherent cell free, second cell culturing support to at least one arrangement position of the plurality of arrangement positions of the first cell culturing support, and
e) culturing the cells on the hybrid cell culturing system, preferably expanding or colonizing the at least one second cell culturing support.

The at least one second cell culturing support is arranged, preferably positioned, to at least one arrangement position, preferably each second cell culturing support is arranged, preferably positioned, to exactly one arrangement position of a plurality of arrangement positions of a first cell culturing support in step a) and/or d) in such a way that a joint surface for cell growth is obtained, wherein the at least one second cell culturing support has direct contact to the first cell culturing support. Thereby, cell migration from the first cell culturing support to the at least one second cell culturing support in order to avoid cell-cell contact inhibition after cell confluence on the first cell culturing support is reached.

In a preferred embodiment, wherein more than one second cell culturing support is arranged to at least one, preferably more arrangement positions of a plurality of arrangement positions of a first cell culturing support, a part of, preferably all second cell culturing supports are removed in step c) from the first cell culturing support.

Preferably, the hybrid cell culturing system used in the process for culturing cells, preferably adherent cells, is specified by one or more technical feature(s) disclosed implicitly or explicitly in connection with the hybrid cell culturing system according to the present invention.

The present invention preferably relates to a process, wherein the steps c) to e) are repeated, preferably at least once, preferably 5 times, preferably 50 times.

Preferably, the cells are seeded on the hybrid cell culturing system prepared in step a) und/or d) before culturing them in step b) und/or e).

Preferably, one second cell culturing support is arranged to each of at least 60%, preferably at least 70%, preferably at least 80%, preferably at least 90%, preferably at least 95%, preferably at least 99%, preferably 100% of the arrangement positions comprised in the first cell culturing support, preferably, to minimize, preferably avoid, cell flow-through and medium outflow in later stages of cultivation.

Preferably, second cell culturing supports exceeding the number of arrangement positions comprised in the first cell culturing support or being not arranged to the arrangement positions of the first cell culturing support are removed prior to step b) and/or e) in order to avoid improper cell attachment or damage to the cells.

Under the term 'cultivation' or 'culturing' any process is understood which can be performed to or by a cell. Preferably, cultivation is understood as attachment of cells, maintaining or increasing cell proliferation, maintaining or increasing cell growth, maintaining or increasing protein expression, maintaining or increasing cell differentiation or two or more cultivation methods thereof. Preferably, cultivation is understood as maintaining or increasing cell proliferation. Preferably, cultivation is understood as maintaining or increasing cell growth.

Preferably, the at least one second cell culturing support is removed in step c) from the first cell culturing support by a mechanical and/or physical method. According to the invention, in step c), the removal of the at least one second cell culturing support is preferably assisted by mechanical or physical methods, in particular by ultrasound, vibrations and/or by rising air bubbles below the porous material. The removal can also be assisted by knocking and/or shaking. Alternatively, the removal is preferably performed by supplying a fluid, preferably a liquid or a gas, preferably air, preferably in a fluid stream, to the at least one second cell culturing support, preferably through the arrangement positions of the first cell culturing support or essentially parallel, preferably parallel to the surface of the first cell culturing support. Preferably, the fluid is applied with a pressure of 1.01 to 4 bar, preferably 1.1 to 3 bar, preferably 1.5 to 2 bar to the support.

Preferably, the at least one second cell culturing support in step c) is removed by flushing or by applying fluid, preferably air pressure, from the opposite site of the at least one second cell culturing support arranged on the first cell culturing support. Thereby, it is possible to restore the cell confluence condition on the first cell culturing support, forcing cells to colonize the newly in step d) arranged, empty at least one second cell culturing support to avoid cell-cell contact inhibition.

Preferably, the at least one second cell culturing support comprises or consists of a superparamagnetic component or mixture of superparamagnetic components. 'Superparamagnetism' is a form of magnetism which appears in small ferromagnetic or ferromagnetic particles. In absence of an internal magnetic field their magnetization appears to be zero in average. However, if an external magnetic field is applied, the particles are magnetized and act like paramagnets. Accordingly, the at least one second cell culturing support is arranged or positioned to/at the at least one, preferably at exactly one, arrangement position of the first cell culturing support by applying a magnetic field to the at least one second cell culturing support so that said at least one second cell culturing support becomes magnetized and is attracted to a magnet which preferably present in the first cell culturing support or on the side of first cell culturing support being opposite to the at least one second cell culturing support. In a preferred embodiment, the at least one second cell culturing support is an iron comprising hydroxyl apatite particle.

In another preferred embodiment of the present invention, the at least one second cell culturing support comprises or consists of at least one paramagnetic component. In this particularly preferred embodiment, a little magnet, preferably in form of a coil, is present at each arrangement position, preferably in the interior of the first cell culturing support. Accordingly, the at least one second paramagnetic cell culturing support is attracted to the at least one arrangement position of the first cell culturing support by the magnet present at, preferably in the interior of, the at least one arrangement position. Preferably, the at least one magnet is positioned on the side of the first cell culturing support, opposite to the at least one first cell culturing support, providing an external magnetic field and/or small magnets, preferably in form of coils, are embedded into the material of the first cell culturing support, preferably providing each a small magnetic field for each arrangement position.

Preferably, the removal in accordance with the present invention is free of any enzymatic reaction. Alternatively or cumulatively, the removal in accordance with the present invention is preferably free of any chemical reaction. Thus, preferably at least 90%, preferably at least 95%, preferably at least 99 %, preferably 100% of the adherent cells attached to the at least one second cell culturing support remain attached thereto when the at least one second cell culturing support is removed in step c) from the first cell culturing support.

Alternatively or cumulatively, the removal in accordance with the present invention is preferably free of any shear stress. Alternatively or cumulatively, the removal in accordance with the present invention is preferably free of any detachment of cells, preferably adherent cells. Alternatively or cumulatively, culturing and passaging in accordance with the present invention is preferably free of any detachment and/or shear stress of cells, preferably adherent cells. Preferably, the removal in accordance with the present invention is free of any enzymatic reaction, chemical reaction, shear stress and detachment of cells, preferably adherent cells.

Preferably, the at least one removed second cell culturing support is transferred to another cell culture device, preferably another first cell culturing support in accordance with the present invention as to allow expansion of the cells, preferably adhering cells, in said further cell culture device, preferably hybrid cell culturing system.

According to the invention, the cells are preferably cultivated in step b) and/or e) on an upwardly directed surface of the hybrid cell culturing system, preferably of the first and/or second cell culturing support. According to the invention, the first surface of the hybrid cell culturing system, preferably first and/or second cell culturing support, is therefore preferably directed upwardly.

If the planar support is preferably used horizontally in accordance with the invention, then, in accordance with the invention, preferably the upper surface of the planar support serves as the growth area of the cells. As the adherent cells adhere to the planar support, the lower surface of the planar support can also serve as the growth area of the cells. The planar support can also be reversed during step b) and/or e) so that, for example, the cells initially grow on the upper surface, the membrane is reversed and the cells thus continue to grow on the lower surface.

The cultivating is preferably carried out in a cell culture medium. The duration of the cultivation, cultivation temperature, cultivation pressure, pH and oxygen content of the medium and other parameters can be selected by a person skilled in the art from the ranges which he is familiar with.

According to the invention, the cells are preferably applied to the hybrid cell culturing system, preferably to first and/or second cell culturing support, left to adhere and then further cultivated.

According to the invention, the cells are preferably applied to the hybrid cell culturing system, preferably to first and/or second cell culturing support, at a cell concentration during seeding of from 4,000 to 20,000 cells/cm², in particular of from 4,000 to approx. 16,000 cells/cm².

According to the invention, the cells suspended in a cell culture medium are at the beginning of step b) and/or e) preferably pipetted drop wise onto the hybrid cell culturing system, preferably to first and/or second cell culturing support, at roughly constant, in particular at constant spacing, using a pipette. During this process, the following parameters are variable for a person skilled in the art: drop volume, drop spacing, cell concentration in the drop and/or waiting time until the first exchange of media. These parameters also allow the cultivation behavior of the respective cells to be altered. According to the invention, the drop volume is preferably between at least 2.5 µl and at most 30 µl. According to the invention, preferably between 10 and 500, in particular between 25 and 350 cells are present in a drop during seeding.

Alternatively, the hybrid cell culturing system, preferably first and/or second cell culturing support, can also be populated in a manner other than the pipetting of droplets, for example by spraying or pouring-over the cells comprised in the cell culture medium.

In a preferred embodiment of the invention, adherent cells are left in step b) and/or e) to adhere to the hybrid cell culturing system, preferably first and/or second cell culturing support, and then further cultivated while adding liquid, in particular cell culture medium.

In a preferred embodiment of the invention, the cells are cultivated in step b) and/or e) until they are almost confluent or until they are confluent.

Provision may also be made to exchange in step b) and/or e) the cell culture medium as required, i.e. to replace spent cell culture medium with fresh cell culture medium.

The term "confluence" is used to describe the closest possible arrangement of adherent cells at the surface of the hybrid cell culturing system. The confluence differs from cell line to cell line.

Shortly before or when confluence is reached as a result of the growing and/or the propagation of the cells, the at least one second cell culturing support is preferably removed according to step c).

The cultivation is preferably controlled by metrological test, for example via electrodes for measuring the TEER value and/or the automated supply of media.

Preferably, step b) and/or e) is ended when the desired cell density is reached, in particular when the cells are almost confluent or are confluent.

By measuring the TEER value (TEER = transepithelial electrical resistance), the density of the cell population can be determined. Thus, it is possible to determine the point in time at which the cells on the hybrid cell culturing system have reached the desired cell density, in particular the point in time at which the cell layer has become almost confluent or confluent.

Preferably, the opacity of the cell culture medium is measured in step b) and/or e). Preferably, the oxygen content of the cell culture medium is measured in step b) and/or e). Preferably, the glucose content of the cell culture medium is measured in step b) and/or e). Preferably, the pH value of the cell culture medium is measured in step b) and/or e).

The present invention relates also to a cell culture device comprising a hybrid cell culturing system according to the present invention.

The present invention preferably relates to a cell culture device according to the present invention, wherein
i) the first cell culturing support is fixed in the cell culture device and
ii) the at least one second cell culturing support is arranged, preferably positioned or immobilized, to at least one arrangement position of the first cell culturing support.

According to the invention, provision is therefore preferably made for the first cell culturing support to be at least partly surrounded by a cell culture device so that the cell culture device can receive a liquid. Preferably, the cell culture device can receive the liquid, which liquid preferably surrounds first cell culturing support, i.e. being located below and/or above the hybrid system.

A broad range of cell culture devices can be used. Cell culture device are preferably selected form cell a culture flask, a cell culture dish, a multiwell plate and a bioreactor, preferably a bioreactor.

According to the invention, the hybrid cell culturing system preferably does not cover the bottom of the cell culture device. According to the invention, the hybrid cell culturing system is preferably located in the interior of the cell culture device, so that there is a free space below and above the hybrid cell culturing system.

In another preferred embodiment of the present invention, the hybrid cell culturing system lies on the bottom or covers the bottom of the cell culture device, so that there is solely a free space above the hybrid cell culturing system. Alternatively, the hybrid cell culturing system is preferably located on the top of the cell culture device, preferably of a compartment of the cell culture device, so that there is solely a free space below the hybrid cell culturing system.

In an alternative embodiment according to the invention provision may be made for the first cell culturing support to be part of a cell culture insert. The cell culture insert can be a conventional cell culture insert from the prior art, such as is offered by various companies.

In a preferred embodiment according to the invention the first cell culturing support is the bottom of a cell culture insert which is inserted in a cell culture vessel, there being a spacing between the bottom of the cell culture device and the first cell culturing support. In step b) of the method according to the preferred embodiment the cells are cultivated in a cell culture medium, the cell culture device being filled with so much cell culture medium that the cell culture medium covers the cells. The cell culture medium can be supplied in step b) in a broad range of ways.

Preferably, the cell culture device has an, in particular closable, opening below the first cell culturing support, through which the fluid for removal of the second cell culturing support can be added. Preferably, the cell culture device has an, in particular closable, opening above the first cell culturing support, through which the cell culture medium can be added or removed.

In an automated method the precise amount of cell culture medium and the fluid for removal of the at least one first cell culturing support can be supplied in a computer-controlled manner in the precisely required amount. In this case, either the amount can be calculated or determined in advance, based on the dimensions of the cell culture vessel and the cell culture insert, or measuring means are provided for measuring the filling level.

According to the invention, a bioreactor for the cultivation of, preferably adherent, cells is preferred, the reactor space of the bioreactor being divided by the first cell culturing support, preferably by the hybrid cell culturing system, into two reactor regions and each of these two reactor regions having an opening suitable for letting in and/or letting out a liquid. In an alternatively preferred embodiment of the present invention, solely one of the two reactor regions have at least one opening suitable for letting in and/or letting out a liquid. Alternatively, a bioreactor is preferred, wherein the first cell culturing support is fixed on the top and/or the bottom of the cell culture device resulting in one reactor region which has at least one opening suitable for letting in and/or letting out a liquid.

The bioreactor surrounds, as the housing, a reactor space, also referred to as the reactor chamber. This reactor space is split up by the first cell culturing support, preferably by the hybrid cell culturing system, into two reactor regions. The bioreactor therefore preferably has two reactor regions or reactor space regions.

The first cell culturing support separates in the hosting bioreactor two different compartments, of which one is intended to be used for the arrangement of the at least one second cell culturing support and culturing of an elected cell type, and the other is intended to be used for medium exchange, removal of the at least one second cell culturing support particles or culturing of an accessory cell type. The two compartments can communicate through the arrangement positions in the first cell culturing support with each other.

According to the invention, the bioreactor preferably contains a reactor lower part, a reactor cover and a first cell culturing support, preferably a hybrid cell culturing system according to the present invention. According to the invention, the bioreactor preferably consists of a reactor lower part, a reactor cover and a membrane unit. According to the invention, the reactor lower part is preferably upwardly opened. The first cell culturing support, preferably the hybrid cell culturing system, then covers the opening in the reactor lower part in that the first cell culturing support, preferably by the hybrid cell culturing system, is positioned horizontally on the opening. The reactor cover then has a downwardly directed opening. The reactor cover is then placed onto the reactor lower part, so that the opening in the reactor cover is also covered by the horizontal first cell culturing support, preferably the horizontal hybrid cell culturing system.

The openings, which are suitable for letting in and/or letting out a liquid, can be used in particular for letting in and/or letting out the cell culture medium and/or the fluid used for removal of the at least one second cell culturing support.

Preferably, a further support, preferably a membrane with differently located, smaller and/or less arrangement positions than the arrangement positions of the first cell culturing support or with no arrangement positions, is positioned on the side of the first cell culturing support, opposite to the at least one second cell culturing support, as to avoid the escape of the cells in said direction, preferably into the lower compartment of the cell culture device.

According to the invention, in step b), the fluid for removal is preferably introduced through the opening, which is suitable for letting in and/or letting out a liquid, in the reactor lower part.

According to the invention, the bioreactor preferably has at least one ventilation opening.

According to the invention, the at least one ventilation opening is preferably provided with a sterile filter.

According to the invention, the bioreactor preferably has at least one pipetting opening.

According to the invention, the first reactor housing part preferably has at least one pipetting opening. According to the invention, the first reactor housing part preferably has a pipetting opening.

According to the invention, metrological devices, which allow the measurement of data, for example for measuring TEER values, are preferably integrated into the bioreactor.

The bioreactor can selectively be used as a stand-alone module or as a component in an overall system.

The present invention also relates to the use of at least one second cell culturing support designed to be arranged to at least one arrangement position of a first cell culturing support, which at least one second cell culturing support is removable from the at least one arrangement position of the first cell culturing support, for culturing cells.

The present invention preferably relates to the subject-matter of the subclaims.

The present invention is illustrated by the following examples.

### Examples

### Example 1: 2D plastic first cell culturing support for Mesenchymal Stem Cells (MSCs) culturing.

A plasma-treated round culturing Petri dish of plastic material with a culturing surface diameter of 35mm is drilled with drilling points of 200µm of diameter in order to produce non protruding arrangement positions of 200µm of diameter spacing 500µm from one another. The arrangement positions produced this way are then cleared from remnants using a sonic bath for half an hour and flushing extensively with water or Phosphate-buffered saline (PBS). The treated Petri dish is then sterilized with gamma-radiation and kept sterile. A bioreactor designed as shown in Figure 1 is used to host the Petri dish, which will be then later considered as the first cell culturing support. The first cell culturing support divides the bioreactor in a lower compartment encased in a lower part, accessible from the outside through a hole allowing liquid or air inflow for second cell culturing support removal, and an upper compartment encased in a upper part, accessible from above in order to allow positioning of the second cell culturing supports, cell seeding, medium exchange and withdrawing of the second cell culturing supports after removal from the first cell culturing support. The bioreactor is closed in the upper part by a removable transparent lid, and the bottom of the bioreactor is made of transparent plastic, allowing observation of the cultured cells under a microscope. The lower and upper part are screwed together in order to seal the culturing chambers, and two O-ring rubber sealings are placed at the interface between the two compartments in order to avoid leaking of the medium from one compartment to the other from the outside and to avoid contamination. The second cell culturing supports are constituted by porous collagen microbeads with an average diameter of 150µm. After swelling in PBS overnight the average diameter of the collagen microbeads is around 200µm. A syringe is connected to the hole in the lower compartment and the lower compartment is filled with culture medium. After removal of the lid, 30mg of microbeads resuspended in 5ml of culture medium are added to the first cell culturing support from the upper chamber and let settle in the arrangement positions. Negative pressure is then applied by sucking culture medium from the lower compartment, immobilizing the microbeads into the arrangement positions of the first cell culturing support. The bioreactor is then slightly inclined and the excess of microbeads is removed from the bioreactor. Mesenchymal Stem Cells (MSCs) are then added to the upper chamber in a small volume in order to avoid detachment of the second cell culturing supports from the arrangement positions. After reaching confluence on the first cell culturing support, the MSCs colonize the second cell culturing supports. The microcarriers are then removed from the first cell culturing support by injecting culture medium in the lower chamber and letting it pass to the upper chamber, bringing the microcarriers in solution. Upon removal of the lid, the microcarriers in solution can be removed and new empty microcarriers can be seeded on the first cell culturing support.

### Example 2: 3D biocompatible polymeric first cell culturing support for fibroblast culturing

A 3D polymeric scaffold of cylindrical shape is processed by freeze drying in order to create aligned arrangement positions with openings on opposite sides of the scaffold, and with salt leaching in order to create arrangement positions in the structure expanding the culturing surface and facilitating diffusion of nutrients to the cells. The diameter of the arrangement position openings created with the freeze drying process is around 400µm. The 3D first cell culturing support is then fixed into a bioreactor divided in three compartments so that the two round surfaces of the cylinder are isolated from the bulk of the scaffold by an O-ring sealing and the distal parts of the scaffold come out into two chambers at the extremes of the bioreactor. The bulk of the scaffold is contained into a central chamber used to perfuse the scaffold with culture medium. The second cell culturing support is constituted by porous microcarriers of spherical shape with an average diameter of 350 µm after swelling. Fibroblasts are loaded to the scaffold by injection and let expanded until the arrangement positions are completely colonized. The scaffold shouldn't be degradable and the arrangement positions created with the salt leaching process shouldn't be big enough to allow migration of the fibroblast outside of the aligned arrangement positions, resulting in full colonization of the aligned arrangement positions and cell confluence therein. The microcarriers are loaded on the scaffold by injection into the aligned arrangement positions while temporarily capping the side opposite to the loading side. After migration of the cells from the first cell culturing support to the RRCS, the microcarriers are removed by flushing with culture medium injected into the chamber opposite to the seeding chamber. After removal of the at least one second cell culturing support, new empty microcarriers are loaded into the scaffold.

### Example 3: Endothelial cells and MSCs co-culture on animal-derived membrane

An animal-derived membrane produced by removing the serosa membrane and the mucosa layer from a decellularized tract of porcine intestine is fixed into a holding device able to keep the membrane in tension. Arrangement positions in the membrane of 400 µm in diameter are produced by pricking the membrane in its central part with a needle. The fixed membrane therefore constitutes the first cell culturing support, and divides the space in the bioreactor in an upper chamber, intended for MSCs culture, and a lower chamber, intended for endothelial cells culture. The upper chamber is accessible from above by removing the lid, similarly to Example 1, while the lower chamber is accessible from the outside by two arrangement positions drilled into diametrically opposite positions, in order to allow automatic perfusion of the medium into the lower chamber. The second cell culturing supports are constituted by porous microcarriers of spherical shape with an average diameter of 400 µm after swelling. After sealing the device, the bioreactor is flipped outside down so that the lower chamber appears to be in the upper position, and endothelial cells are seeded on the membrane and let grow for some days under perfusion. After significant growth of the endothelial cells on the future lower side of the membrane, the bioreactor is flipped to the original position and the microcarriers are seeded on the non-colonized side of the membrane and the excess of microcarriers is removed. The MSCs are then seeded by injection in the upper chamber and let grow until confluence on both the second cell culturing supports and the first cell culturing support. The second cell culturing supports are then removed by tapping one of the holes in the lower chamber and injecting culture medium in the remaining arrangement position, bringing the colonized microcarriers in solution. The microcarriers can then be collected from above after removal of the lid.

### Example 4 - Protocol for Bioreactor System: Expansion of bone marrow derived mesenchymal stem cells (BM-hMSC) on collagen-based microcarriers

A Bioreactor System is constituted by a bioreactor in which two compartments of circular shape are screwed together in order to fix the first culturing surface. The first culturing surface is a commercially available Petri dish of 3.5cm of internal diameter which has been drilled under non-sterile conditions with an automatic drilling machine in order to create pores through the whole depth of the Petri dish and characterized by a diameter of 200µm and a spacing between the pores of 700µm from the center of the pore. The two compartments of the bioreactor comprise each a hollow chamber. After the assembling of the bioreactor, the chambers communicate through the pores of the first culturing surface, which is fixed between the two compartments and isolated through an O-ring sealing. The bottom compartment presents two holes on the sides for tubing connections allowing in- and out- flow of the culture medium through a syringe. The bottom compartment presents a glass window allowing microscopical inspection of the culturing surface. The top compartment is characterized by the presence of a large hole able to host the lid of the Petri dish, which can be removed to allow medium exchange and beads seeding and withdrawal.

After assembling of the bioreactor, the protocol proceeds as follows:
1. 20 ml of DMEM-F12 (Dulbecco's Modified Eagle Medium/Nutrient Mixture F-12) 10% FCS (fetal calf serum) are added to the upper chamber upon removal of the lid of the Petri dish;
2. The Medium is sucked into the lower compartment through application of negative pressure on the syringes;
3. Eventual air bubbles are removed from the lower compartment by sucking with a pipette accessing the bioreactor from the upper compartment;
4. 30mg of collagen-based microbeads with an average diameter of 210µm after swelling are diluted in 500µl of DMEM-F12 10% FCS and added to the culturing surface;
5. The beads are sucked into the pores of the Petri dish by applying negative pressure through the syringes;
6. Excess of beads is removed by inclining the bioreactor and aspirating the beads deposited on the side of the culturing surface;
7. 10^5 BM-hMSCs diluted in 500µl of DMEM-F12 10% FCS are seeded in the bioreactor and allowed to attach to both the culturing surface and the collagen-based microcarriers;
8. The cells are let grow on the hybrid system until confluence, changing half of the medium from the upper compartment every 24h;
9. After confluence is reached, 10ml of medium is pumped from the two syringes on the bottom compartment, allowing detaching of the collagen-based microcarriers from the culturing surface;
10.The collagen-based microcarriers are collected with a 10ml glass pipette;
11.The protocol is repeated from point 4 to point 10 avoiding point 7, and cell behavior is monitored over time.

Upon removal of the cell-colonized collagen-based microcarriers from the bioreactor, viability of the cells is assessed through DAPI (4',6-diamidino-2-phenylindole) and Live/Dead stainings according to the protocols provided by the provider; cell number is established by DNA-content quantification with PicoGreen according to the protocol provided by the provider; and cell morphology is evaluated by SEM (Scanning Electron Microscopy).

Result Perspectives: DAPI and Live/Dead Stainings reveal good colonization of the microcarriers after the second passaging cycle; DNA content quantification reveals constant cell number on colonized beads, with negligible decrease over time up to 10 population doublings; SEM analysis reveals optimal cell morphology when compared to beads colonized in spinner flask systems.

## Claims

1. A hybrid cell culturing system comprising:
i) a first cell culturing support fixable in a cell culture device, which first cell culturing support comprises a plurality of arrangement positions, and
ii) at least one second cell culturing support designed to be arranged to at least one arrangement position of the first cell culturing support, which at least one second cell culturing support is removable from the at least one arrangement position of the first cell culturing support.

2. The hybrid cell culturing system according to claim 1, wherein the shape and dimension of the at least one second cell culturing support allows the at least one second cell culturing support to be arranged at least partially in or on the at least one arrangement position of the first cell culturing support.

3. The hybrid cell culturing system according to any one of the preceding claims, wherein the arrangement positions of the first cell culturing support have an average diameter from 1 to 550 µm.

4. The hybrid cell culturing system according to any one of the preceding claims, wherein the at least one second cell culturing support has a diameter from 1 to 600 µm.

5. The hybrid cell culturing system according to any one of the preceding claims, wherein the first cell culturing support is a planar or three-dimensional support.

6. The hybrid cell culturing system according to any one of the preceding claims, wherein the planar support is a Petri dish, a plate or a membrane.

7. The hybrid cell culturing system according to any one of the preceding claims, wherein the three-dimensional support is a polymeric scaffold, an organ or a tissue.

8. The hybrid cell culturing system according to any one of the preceding claims, wherein the at least one second cell culture support is porous or non-porous.

9. A process for culturing cells using a hybrid cell culturing system according to any one of the preceding claims.

10. A process for culturing cells, comprising the following process steps:
a) arranging at least one second cell culturing support to at least one arrangement position of a plurality of arrangement positions of a first cell culturing support, as to obtain a hybrid cell culturing system, and
b) culturing cells on the hybrid cell culturing system.

11. The process according to claim 10, wherein after step b) the following steps are performed:
c) removing the at least one second cell culturing support from the first cell culturing support,
d) arranging at least one further second cell culturing support to at least one arrangement position of the plurality of arrangement positions of the first cell culturing support, and
e) further culturing the cells on the hybrid cell culturing system obtained in step d) and preferably colonizing of the at least one second cell culturing support.

12. The process according to claim 11, wherein steps c) to e) are repeated.

13. A cell culture device comprising a hybrid cell culturing system according to any one of the claims 1 to 8.

14. The cell culture device according to claim 13, wherein
i) the first cell culturing support is fixed in the cell culture device and
ii) the at least one second cell culturing support is arranged to at least one arrangement position of the first cell culturing support.

15. Use of at least one second cell culturing support designed to be arranged to at least one arrangement position of a first cell culturing support, which at least one second cell culturing support is removable from the at least one arrangement position of the first cell culturing support, for culturing cells.
